# EUROPÄISCHE PATENTSCHRIFT

(11) **EP 0 688 789 B1**
(45) Veröffentlichungstag und Bekanntmachung des Hinweises auf die Patenterteilung: **19.06.2002**
(21) Anmeldenummer: 95104376.9
(22) Anmeldetag: 24.03.1995
(51) Int. Cl.: C07K 7/60, A61K 38/12

(54) **Lipopeptid-Derivate, Verfahren zu ihrer Herstellung und ihre Verwendung**
Lipopeptid-derivatives, process for their preparation and their use
Dérivés des lipopeptides, procédé de leur préparation et leur utilisation

(30) Priorität: 30.03.1994 DE 4411025
(43) Veröffentlichungstag der Anmeldung: 27.12.1995
(73) Patentinhaber: Aventis Pharma Deutschland GmbH, 65929 Frankfurt am Main (DE)
(72) Erfinder: Lattrell, Rudolf, Dr., D-61462 Königstein (DE); Wollmann, Theodor, Dr., D-65719 Hofheim (DE); Wallmeier, Holger, Dr., D-65843 Sulzbach (DE); Hammann, Peter, Dr., D-64832 Babenhausen (DE); Isert, Dieter, Dr., D-65760 Eschborn (DE)

(56) Entgegenhaltungen:
- EP-A- 0 629 636
- J.ANTIBIOT., Bd. 22, Nr. 10, 1969 Seiten 473-79, JJ.SHOJI ET AL. 'Studies on Tsushimycin. II The Sztructures of Constituent Fatty Acids'
- ANTIMICROB.AG.CHEMOTHER., 1970 Seiten 42-45, R.C.STRONG 'Studies on the Amino Acid Sequence of Amphomycin'

## Beschreibung

Die Erfindung betrifft Derivate von Antibiotika des Lipopeptidkomplexes A 1437, ein Verfahren zu ihrer Herstellung und ihre Verwendung als Arzneimittel.

In der EP 0 629 636 werden Lipopeptide mit sehr homologen Aminosäuresequenzen, jedoch unterschiedlichen Fettsäureresten (Lipidanteil) vorgeschlagen, die von Actinoplanes sp. während der Fermentation synthetisiert und in das Kulturmedium abgegeben werden sowie ein Verfahren zur Isolierung der Lipopeptide aus dem Kulturmedium, ihre Aufreinigung und die Verwendung der Lipopeptide als pharmakologische Wirkstoffe, insbesondere gegen gram-positive Bakterien.

Strong et al. offenbaren in "Antimicrobial Agents and Chemotherapy, 1971, Seiten 42-45" die Antibiotika Amphomycin, Gluamycin und Tsushimycin. Die drei Verbindungen sind cyclische Peptidantibiotika mit charakteristischer Aminosäurezusammensetzung, die sich aus Tabelle 1 des Dokuments ergibt.

Shoji et al. offenbaren in "The Journal of Antibiotics 22, 473-479, 1969" Tsushimycin II enthaltend als Fettsäurekomponente cis-3-Anteisopentadecensäure.

Aufgabe dieser Erfindung ist es nun, nach Derivaten des Lipopeptidkomplexes A 1437 mit einer geringeren Toxizität im Vergleich zu den natürlichen A 1437 Lipopeptiden zu suchen.

Diese Aufgabe wird erfindungsgemäß gelöst durch Derivate gemäß einer Verbindung der Formel I.

Die Erfindung betrifft somit:
1. Lipopeptid A 1437-Derivate der Formel I, 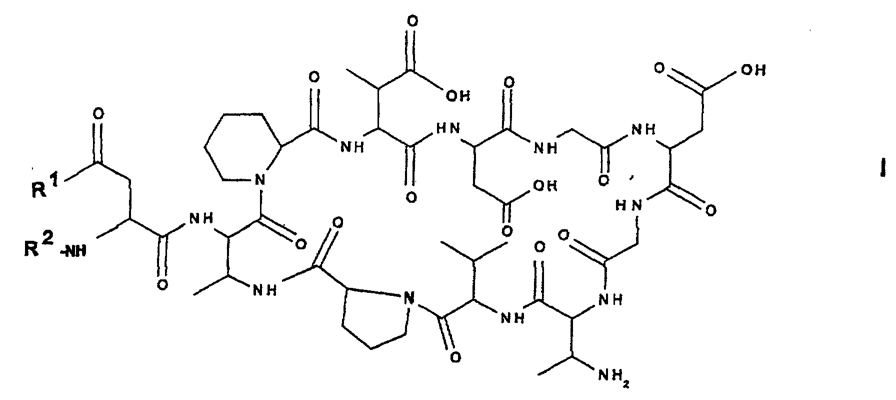 worin
   - R¹: OH oder NH₂,
   - R²: ein geradkettig oder verzweigter gesättigter oder ungesättigter aliphatischer C₈-C₂₂-Acylrest ist, der durch Phenyl- oder Cycloalkylgruppen oder durch Sauerstoff unterbrochen ist,
   und worin n eine ganze Zahl zwischen 0 und 20 bedeutet
   und deren pharmazeutisch verträgliche Salze.
2. Verfahren zur Herstellung einer Verbindung der Formel I, dadurch gekennzeichnet, daß man eine Verbindung der Formel II 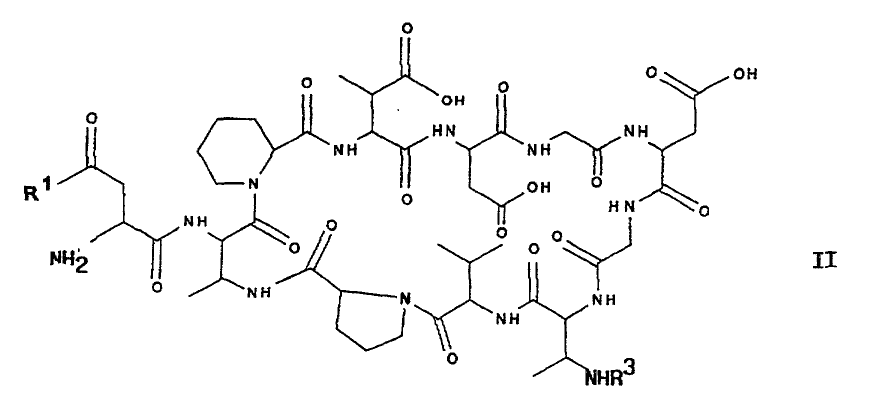 worin
   - R¹: die oben genannte Bedeutung hat und
   - R³: eine aus der Peptidchemie bekannte Aminoschutzgruppe, vorzugsweise die tert.-Butoxycarbonyl (BOC-), die Benzyloxycarbonyl- (Z-, Cbz-), die Fluorenylmethoxycarbonyl (Fmoc-) oder die Allyloxycarbonyl- (Alloc-) Schutzgruppe bedeutet,
   mit einer Carbonsäure der Formel III,

   R²OH III

   worin
   - R²: die in Anspruch 1 genannten Bedeutungen hat
   oder mit einem an der Carbonylgruppe aktivierten Derivat dieser Carbonsäure umsetzt.
3. Arzneimittel, enthaltend ein Lipopeptid-Derivat gemäß Formel I sowie pharmazeutische Träger.
4. Verwendung eines Lipopeptid-Derivats gemäß Formel I zur Herstellung eines Arzneimittels gegen bakterielle Infektionen.

Im folgenden wird die Erfindung detailliert beschrieben, insbesondere in ihren bevorzugten Ausführungsformen. Ferner wird die Erfindung durch den Inhalt der Patentansprüche bestimmt.

Die Ausgangsverbindungen (Verbindungen der Formel II) werden aus den geschützten Fermentationsprodukten erhalten, z.B. aus A 1437 β (I, R¹=OH, R²=(CH₃)₂CH(CH₂)₇CH=CHCH₂CO) und Chlorameisensäure-(9-fluorenylmethyl)ester unter Bildung der entsprechenden Verbindung mit 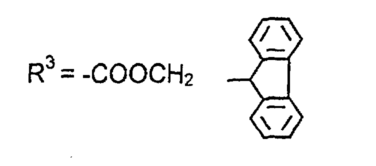 und nachfolgender enzymatischer Abspaltung des Fettsäurerestes mittels Actinoplanes utahensis NRRL 12052 (J. Antibiotics 1988, 1093).

Werden die Carbonsäuren der Formel III selbst als Acylierungsmittel eingesetzt, so wird zweckmäßig in Gegenwart eines Kondensationsmittels, z. B. eines Carbodiimids wie N,N'-Dicyclohexylcarbodiimid gearbeitet. Die Aktivierung der Carbonsäuren der Formel II kann nach den in der Peptidchemie üblichen Methoden wie sie z. B. in "Chemie in unserer Zeit" 27, 274-286 (1993) beschrieben sind, erfolgen. Als aktivierte Derivate eignen sich demnach Säurehalogenide, z. B. Säurechloride, Anhydride oder gemischte Anhydride,
z.B. mit Ameisensäureestern, Azide, aktivierte Ester wie p-Nitrophenyl-, Pentafluorphenyl-, 4,6-Dimethoxy-1,3,5-triazin-2-yl-Ester oder Ester mit N-Hydroxysuccinimid oder 1-Hydroxybenzotriazol, die mit Carbodiimiden als Kupplungsreagenzien erhalten werden, oder Thioester, z.B. mit 2-Mercaptobenzotriazol. Weitere geeignete Kupplungsreagenzien sind N,N'-Carbonyldiimidazol oder solche auf der Basis von Phosphonium- oder Uroniumsalzen wie z. B. BOP, HBTU, PyBOP, TBTU oder TOTU (O-[Cyano(ethoxycarbonyl)methylidenamino-1,1,3,3-tetramethyl]uroniumtetrafluoroborat).

Im allgemeinen erfolgt die Umsetzung der Verbindungen der Formel II mit einer Carbonsäure der Formel III oder deren aktiviertem Derivat in Gegenwart eines inerten Lösungsmittels wie z. B. Dichlormethan oder Dimethylformamid, vorzugsweise in Gegenwart einer tertiären Base wie z.B. Pyridin oder Ethyldiisopropylamin. Bei Verwendung von substituierten Benzoylchloriden kann auch in Gegenwart von Wasser und Zusatz von Basen wie Pyridin oder Natriumcarbonat gearbeitet werden. Die Umsetzung kann in einem Temperaturbereich von -20° bis + 50°, vorzugsweise zwischen -10° und +30°C erfolgen.

Die Abspaltung der Schutzgruppen R³ unter Bildung der Verbindungen I erfolgt nach literaturbekannten Verfahren, z.B. wird die BOC-Gruppe mit Trifluoressigsäure, der Z-Rest mit HBr/Eisessig oder durch katalytische Hydrierung, die Alloc-Gruppe mit Nucleophil plus Pd- Katalysator oder die Fmoc-Gruppe mit sekundären Aminen, z.B. Piperidin, abgespalten.

Bevorzugt sind die Verbindungen der Formel I, in den R² ein durch Phenyl- oder Cycloalkylreste unterbrochener aliphatischer Acylrest, vorzugsweise 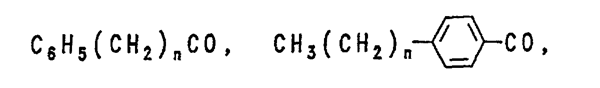 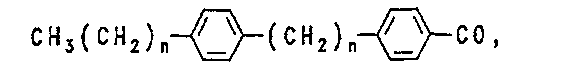 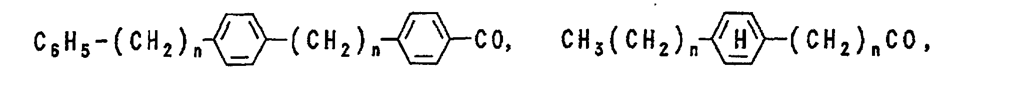 durch einen Sauerstoff unterbrochener Acylrest, vorzugsweise 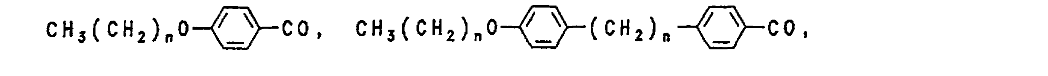 und worin n ganze Zahlen zwischen 0 und 20 bedeutet.

Besonders bevorzugt sind die Verbindungen, die einen durch 1-3 Phenylreste und/oder zusätzlich durch Sauerstoff unterbrochenen aliphatischen Acylrest, wie z.B. 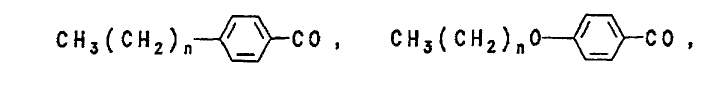 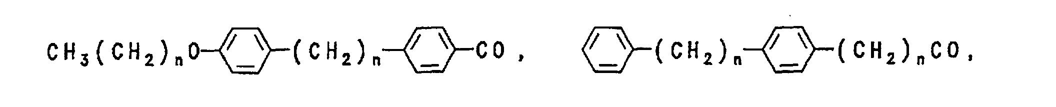 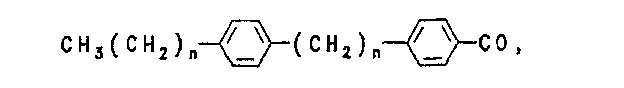 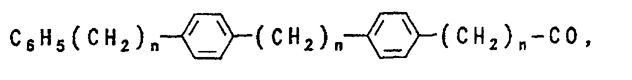 worin n ganze Zahlen zwischen 0 und 8 bedeuten.

Ganz besonders bevorzugt sind die Verbindungen, die einen durch 3 Phenylgruppen unterbrochenen aliphatischen Acylrest, wie z.B. 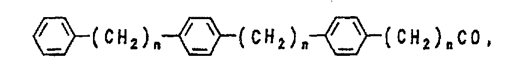 worin
n ganze Zahlen zwischen 0 und 2 bedeuten, enthalten.

Die Erfindung umfaßt weiterhin ein Verfahren zur Herstellung von Verbindungen der Formel I, das dadurch gekennzeichnet ist, daß man
eine Verbindung der Formel II 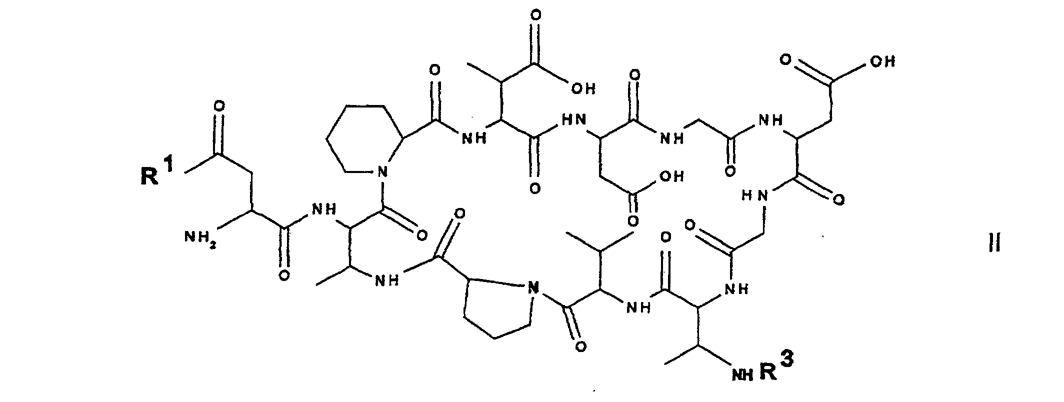 worin
- R¹: die oben genannte Bedeutung hat und
- R³: eine aus der Peptidchemie bekannte Aminoschutzgruppe, wie z. B. die tert.-Butoxycarbonyl (BOC-), die Benzyloxycarbonyl- (Z-, Cbz-), die Fluorenylmethoxycarbonyl (Fmoc-) oder die Allyloxycarbonyl- (Alloc-) Schutzgruppe bedeutet,
mit einer Carbonsäure der Formel III,

R²OH III

worin R² die schon genannten Bedeutungen hat,
umsetzt.

Als pharmazeutisch verträgliche Salze der Verbindungen der Formel I sind Salze mit anorganischen und organischen Säuren, z.B. Salzsäure, Schwefelsäure, Essigsäure , Citronensäure, p-Toluolsulfonsäure, mit anorganischen und organischen Basen wie NaOH, KOH, Mg(OH)₂, Diethanolamin, Ethylendiamin oder mit Aminosäuren wie Arginin, Lysin, Glutaminsäure u.s.w. besonders nützlich. Sie werden nach Standardvorschriften hergestellt.

Eine oder mehrere Verbindungen der erfindungsgemäßen Lipopeptide bzw. ihre Salze eignen sich aufgrund ihrer wertvollen pharmakologischen Eigenschaft zur Anwendung als Arzneimittel.

Die erfindungsgemäßen Substanzen besitzen pharmakologische Wirksamkeit insbesondere als Antibiotikum gegen gram-positive Bakterien, besonders bevorzugt gegen MRSA- und Glycopeptid-resistente Stämme.

Bei Penicillin- bzw. Methicillin-resistenten Stämmen (MRSA-Stämmen), die weitere Resistenzen gegen Antibiotika ausgebildet haben, besitzen häufig nur Glycopeptide wie Vancomycin oder Teicoplanin eine therapeutisch ausreichende Wirkung. Zunehmend treten jedoch auch gegen diese Antibiotika resistente Stämme auf (FEMS Microbiol. Lett. 98 (1992) 109 bis 116). Ein oder mehrere Verbindungen der erfindungsgemäßen Lipopeptide besitzen eine hervorragende Wirkung auch gegen diese Problemkeime.

Die Erfindung betrifft auch pharmazeutische Zubereitungen einer oder mehrerer Verbindungen der erfindungsgemäßen Lipopeptide bzw. ihrer Salze.

Ein oder mehrere Verbindungen der erfindungsgemäßen Lipopeptide, bevorzugt ein oder mehrere Verbindungen mit drei-Phenylradikalen im Acylrest R² können grundsätzlich als solche in Substanz verabreicht werden. Bevorzugt ist die Verwendung in Mischung mit geeigneten Hilfsstoffen, Trägermaterial oder Verdünnungsmitteln. Als Trägermaterial können bei Tierarzneimitteln die üblichen Futtermittelmischungen bzw. beim Menschen alle pharmakologisch verträglichen Trägermaterialien und/oder Hilfsstoffe verwendet werden.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Als Verdünnungsmittel seien beispielsweise erwähnt Polyglykole, Ethanol und Wasser. Puffersubstanzen sind beispielsweise organische Verbindungen, wie z.B. N,N'-Dibenzylethylendiamin, Diethanolamin, Ethylendiamin, N-Methylglucamin, N-Benzylphenethylamin, Diethylamin, Tris(hydroxymethyl)aminomethan, oder anorganische Verbindungen, wie z.B. Phosphatpuffer, Natriumbicarbonat, Natriumcarbonat. Es ist auch möglich, die Wirkstoffe als solche ohne Träger- oder Verdünnungsmittel in geeigneter Form zu applizieren. Geeignete Dosen der Verbindungen der Formel I oder ihrer pharmazeutisch verträglichen Salze liegen bei etwa 0,4 g vorzugsweise 0,5 g bis maximal 20 g pro Tag für einen Erwachsenen von etwa 75 kg Körpergewicht. Es können Einzel- oder im allgemeinen Mehrfachdosen verabreicht werden, wobei die Einzeldosis den Wirkstoff in einer Menge von etwa 50 bis 1000 mg enthalten kann.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Lipopeptide enthält. Bei festen Dosierungseinheiten wie Tabletten, Kapseln und Suppositorien kann diese Dosis bis zu etwa 200 mg, bevorzugt jedoch etwa 0,1 bis 100 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, daß man ein oder mehrere Verbindungen der erfindungsgemäßen Lipopeptide mit üblichen Trägersowie gegebenenfalls Zusatz- und/oder Hilfstoffe in die bzw. eine geeignete Darreichungsform bringt.
Die besonders bevorzugten Verbindungen der Formel I mit 3 Phenylradikalen im Acylrest R² (z.B. 55, 56) besitzen darüber hinaus besonders günstige toxikologische Eigenschaften. So zeigen sie im Standard-Hämolysetest praktisch keine Hinweise auf Hämolyse während alle getesteten Verbindungen mit geradkettigen oder verzweigten aliphatischen Acylresten einschließlich der Naturstoffe eine beträchtliche Aktivität zwischen 16 und 25 % aufweisen (Tabelle 1).

**Tabelle 1:**

| Hämolytische Aktivität in-vitro²⁾ | |
|---|---|
| Beispiel | Hämolyse (%) |
| *A 1437*¹⁾ X CF₃CO₂H | 17.5 |
| 1 | 19.6 |
| 6 | 16.5 |
| 7 | 25.7 |
| 8 | 19.3 |
| 9 | 22.8 |
| 14 | 22.9 |
| 49 | 0.0 |
| 55 | 0.5 |
| 56 | 0.4 |

| | |
|---|---|
| 1) Fermentationsprodukt I (R¹=OH, R²=(CH₃)₂CH(CH₂)₇CH=CHCH₂CO) | |
| 2) Zur Messung der hämolytischen Aktivität wird frisch entnommenes, venöses Blut vom Rhesusaffen verwendet. Das Blut wird in heparinsierten Röhrchen gesammelt und in Aliquots von 200 µl auf 12 Polyethylenröhrchen verteilt. Ein Aliquot wird mit 200 µl destilliertem Wasser versetzt und dient als 100 % Standard, ein anderes wird mit 200 µl physiologischer Kochsalzlösung (0.9 % NaCl) gemischt (0 % Standard). Je 200 µl von Substanzverdünnungen in physiologischer Kochsalzlösung zu 1600, 800, 400, 200, 100, 50, 25, 12.5, 6.25 und 3.125 mg/l werden auf die übrigen Röhrchen verteilt. Sämltiche Röhrchen werden vorsichtig geschwenkt und dann für 3 Stunden bei 37°C inkubiert. Anschließend wird der 100 % Standard mit 5 ml destilliertem Wasser, die übrigen mit je 5 ml physiologischer Kochsalzlösung aufgefüllt und 5 Minuten bei 700 g zentrifugiert. | |

Die Hämolyse wird durch Messung der Absorption des Überstandes in einem Spektralphotometers bei einer Wellenlänge von 540 nm bestimmt. Die Absorption des Standards mit kompletter Hämolyse (Aqua dest.) wird als 100 % gesetzt. Die Absorptionen der Testpräparatverdünnungen und des 0 % Standards werden gemessen und als Prozent maximal induzierbaren Hämolyse angegeben.

Die folgenden Ausführungsbeispiele für erfindungsgemäß herstellbare Verbindungen dienen zur weiteren Erläuterung der Erfindung.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

Die Reinheit der Reaktionsprodukte wird mittels analytischer HPLC (Merck, Darmstadt, LiChrospher(R) 100RP-8, 125 x 4 mm, Elutionssystem Wasser + Trifluoressigsäure pH 2.5, 0,1 % Natriumoctansulfonat/Acetonitril, Detektion mit UV bei 220 nm) bestimmt, die Struktur mittels Elektrospray-Massenspektroskopie (BIO-Q-MS) bewiesen.

Zur Vereinfachung wird im folgenden der Term A 1437-Cyclopeptid für die Verbindung I mit R² = Wasserstoff gebraucht.

### Beispiel 1

### Tridecanoylderivat von A 1437-Cyclopeptid

### (Verbindung I, R¹ = HO, R² = CH₃(CH₂)₁₁CO)

### TOTU-Kupplungsverfahren:

a) Aktivierung der Tridecansäure:
   113 mg (0.527 mmol) Tridecansäure werden in 3.75 ml N,N-Dimethylformamid (DMF) gelöst, 172.5 mg (0.526 mmol) TOTU und 1.25 g einer Lösung von Ethyldiisopropylamin (0.5 mmol) in DMF (0.4 mmol/g) zugegeben. Die Lösung wird 1 Stunde bei Raumtemperatur belassen.
b) Kupplung:
   348 mg (0.264 mmol) Fmoc-Derivat II (R¹ = OH, R³ = Fluorenylmethoxycarbonyl; Beispiel 69) werden in 7.2 ml trockenem DMF suspendiert und 2.9 g der Aktivlösung a) (0.25 mmol) im Eisbad zugegeben. Die gebildete bräunliche Lösung wird 1.5 Stunden bei Raumtemperatur gerührt.
c) Abspaltung der Fmoc-Schutzgruppe:
   Die Lösung b) wird auf 10° gekühlt, 6 ml Piperidin zugegeben und 1 Stunde bei Raumtemperatur gerührt. Sodann wird mit 250 ml Wasser verdünnt und gefriergetrocknet.
d) Reinigung:
   Der gefriergetrocknete Rückstand wird in 100 ml Wasser/Acetonitril (5:1) suspendiert, mit 1.5 ml 2N HCI auf pH 2.0 gestellt und die klare Lösung über 90 g RP₁₈-Kieselgel (Merck, Art. 9303) mit Wasser + 0.01 %
   CF₃COOH/Acetonitril chromatographiert. Elutionsfolge: 500 ml 3:1, 500 ml 2:1, 600 ml 1:1-Gemisch. Die Titelverbindung erscheint in der 1:1-Fraktion (UV-Detektion 220 nm). Ausbeute 267 mg (78 % d.Th.) Reinheit 72 %.
   Das Rohprodukt wird über eine Büchi-Mitteldrucksäule rechromatographiert (250 g RP₁₈, Elution mit Wasser + 0.01 % CF₃COOH/Acetonitril (3:2)). Die Produktfraktionen werden gefriergetrocknet.
   Ausbeute: 130 mg, Reinheit: 96 %
   C₅₈H₉₃N₁₃O₂₀ [1292.5] MS: 1293

### Beispiel 2

### 4-Octylbenzoylderivat von A 1437-Cyclopeptid

(Verbindung I, R¹ = HO,

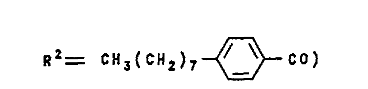

### Säurechloridverfahren:

a) Kupplung:
   6.6 mg (0.005 mmol) Fmoc-Derivat II (Beispiel 69) werden in 200 mg Pyridin/Wasser (9:1) gelöst und bei -20°C 25 mg (0.1 mmol) 4-Octylbenzoylchlorid zugegeben. Die Lösung wird 4 Stunden bei Raumtemperatur gerührt. Nach Zugabe von 2 ml Dioxan wird das Lösungsmittel im Vakuum entfernt und der Rückstand in 0.2 ml DMF gelöst.
b) Abspaltung der Fmoc-Schutzgruppe:
   Zur Lösung a) werden 0.2 ml Piperidin zugegeben und 1 Stunde bei Raumtemperatur belassen. Die Lösung wird mit 5 ml Wasser verdünnt und gefriergetrocknet.
c) Reinigung:
   Der gefriergetrocknete Rückstand wird über 10 g RP₁₈ Kieselgel mit Wasser + 0.01 % CF₃COOH/Acetonitril chromatographiert. Elutionsfolge: 80 ml 3:1, 80 ml 2:1, 80 ml 1:1-Gemisch. Die 1:1-Produktfraktion wird gefriergetrocknet.
   Ausbeute: 4.6 mg (70 % d.Th.), Reinheit: 85 %
   C₆₀H₈₉N₁₃O₂₀ [1312.5] MS: 1313

Analog Beispiel 1 werden die nachfolgend aufgeführten Verbindungen der Formel I erhalten, worin R¹ = HO bedeutet und die den in Tabelle 2 angegebenen Substituenten R² tragen. Die Ausbeuten liegen zwischen 60 und 85 % d.Th., die Reinheit zwischen 75 und 98 %.

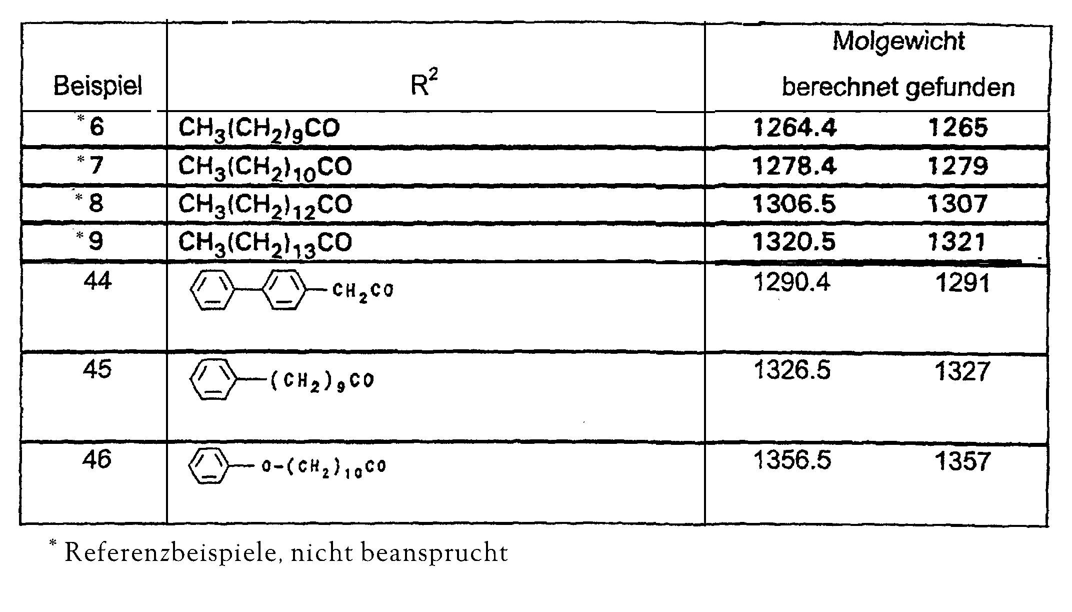

Analog Beispiel 2 werden die nachfolgend aufgeführten Verbindungen der Formel I erhalten, worin R¹ = HO bedeutet und die den in Tabelle 3 angegebenen Substituenten tragen. Die Ausbeuten liegen zwischen 70 und 85 % d.Th., die Reinheit zwischen 80 und 98 %.

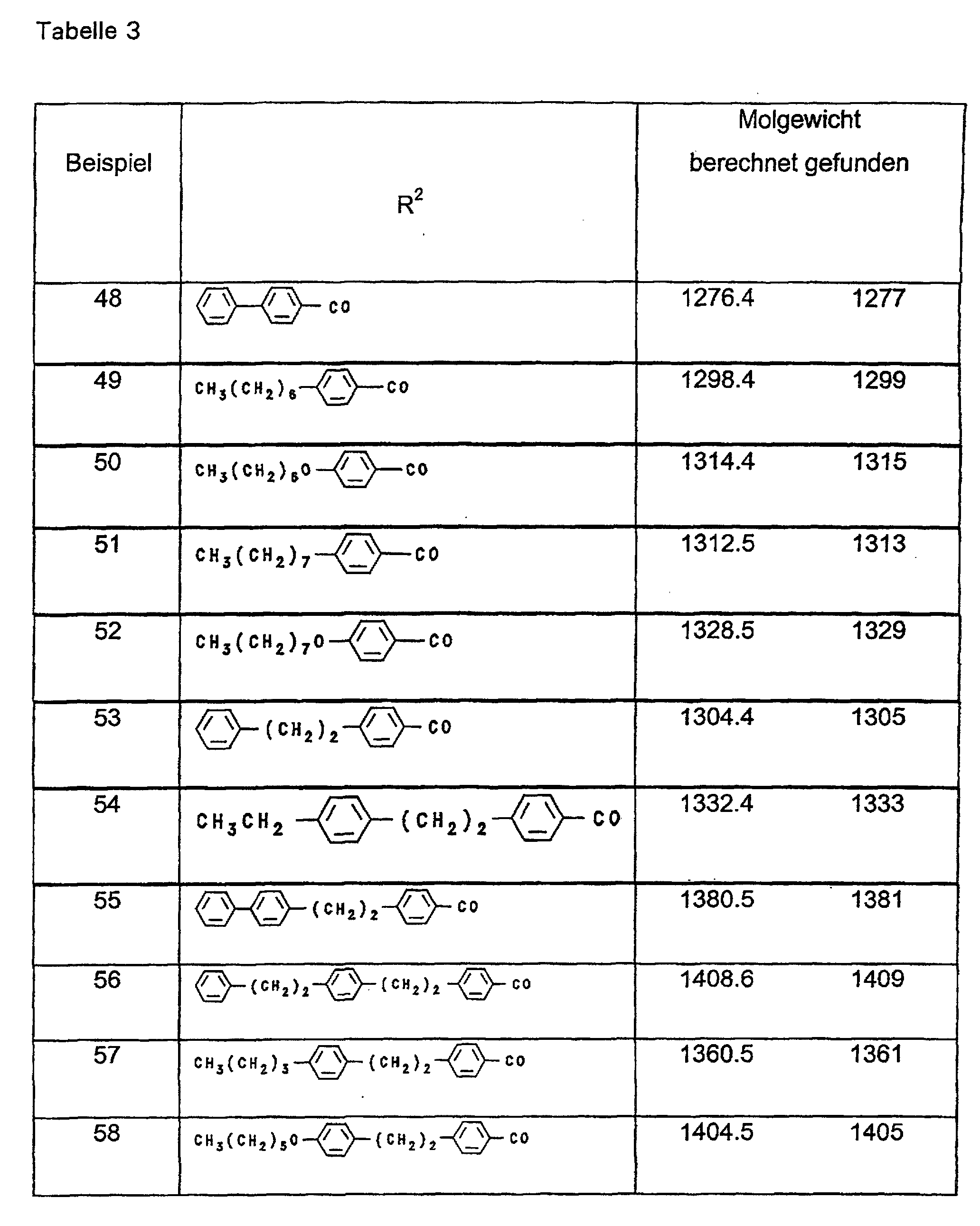

Analog Beispiel 2 (Verbindungen 67 und 68) werden die nachfolgend aufgeführten Verbindungen der Formel I erhalten, worin R¹ NH₂ bedeutet und die den in Tabelle 4 angegebenen Substituenten R² tragen. Die Ausbeuten liegen zwischen 75 und 85 % d.Th. die Reinheit zwischen 80 und 98 %.

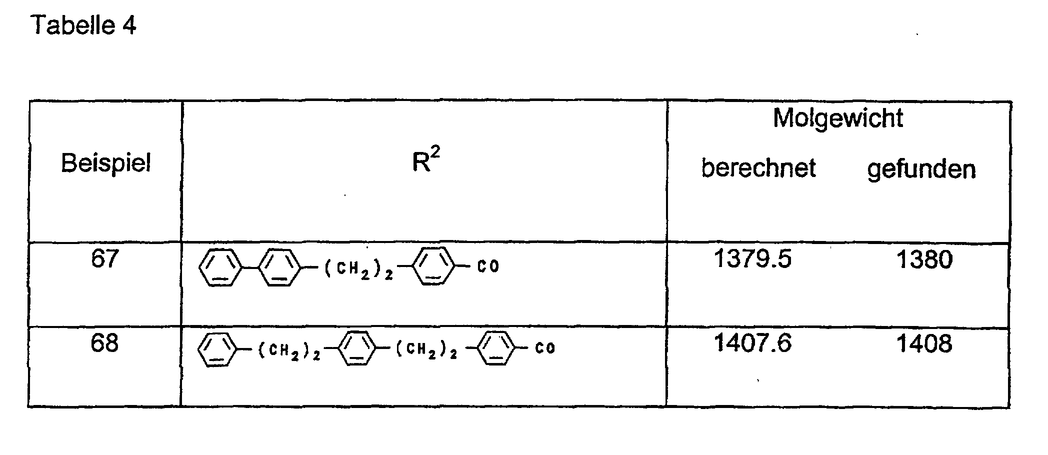

### Herstellung der Ausgangsverbindungen

### Beispiel 69

### 9-Fluorenylmethyloxycarbonyl-Derivat von A 1437

### (R¹ = HO, R² = (CH₃)₂CH(CH₂)₇CH=CHCH₂CO, R³ = -COOCH₂

10 g (7.67 mmol) A 1437 (I, R¹ = HO, R² = (CH₃)₂CH(CH₂)₇CH=CHCH₂CO) und 3.24 g (38.35 mmol) Natriumbicarbonat werden in einem Gemisch aus 920 ml Wasser und 640 ml Aceton gelöst. Unter pH-Kontrolle wird sodann bei pH 8.5 eine Lösung von 2.97 g (11.5 mmol) Chlorameisensäure-(9-fluorenylmethyl)-ester in 240 ml Aceton innerhalb 100 Minuten zugetropft. Hierbei erwärmt sich die Reaktionslösung auf 27°C. Es wird 1 Stunde bei Raumtemperatur nachgerührt. Nach Entfernen des Acetons im Vakuum wird die wäßrige Lösung gefriergetrocknet. Der farblose Rückstand wird zur Entfernung von niedermolekularen Verunreinigungen zweimal mit je 500 ml Methylendichlorid ausgerührt.
Ausbeute: 12.2 g, MS: 1526.7

### Beispiel 70

### 9-Fluorenylmethyloxycarbonyl-Derivat von A 1437-Cyclopeptid (II, R¹ = HO,

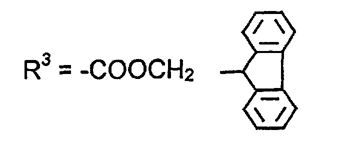

Eine Mischung aus 10 g Produkt aus Beispiel 69 und 300 g feuchtem Mycel aus Actinoplanes utahensis in 1 I sterilem Kaliumphosphatpuffer (100 mmol, pH 7.2, 50 mmol EDTA, 0.02 % Natriumazid) wird 48 Stunden bei 32°C gerührt. Sodann wird die Biomasse durch Zentrifugation abgetrennt, die Lösung zur Fixierung des Produkts über 500 g MCI-Gel (Fa. Mitsubishi) filtriert und das Produkt mit Wasser/Methanol (1:1) eluiert. Das Eluat wird zur Entfernung von Methanol eingeengt und die wäßrige Lösung über 500 g RP₁₈ mit Wasser + 0.05 % Trifluoressigsäure/Acetonitril (2:1) chromatographiert. Die Produktfraktionen werden im Vakuum eingeengt und gefriergetrocknet.
Ausbeute: 6 g, MS: 1318.4

### Beispiel 71

### 4-((2-(4-(2-Phenylethyl))phenylethyl))benzoesäure

Eine Lösung von 22.9 g 4-Brommethylbenzoesäuremethylester in 1000 ml Toluol wird mit 33.9 g Triphenylphosphin versetzt und unter Rückfluß erhitzt. Nach 7 Stunden ist die Umsetzung vollständig. Man läßt abkühlen und saugt das Produkt ab.
Ausbeute: 47.6 g

### Stufe 2

58.9 g Stufe 1 werden in 500 ml wasserfreiem Tetrahydrofuran suspendiert, auf 0°C gekühlt und mit 120 ml einer 1 M Lösung von Lithium-bis-trimethylsilylamid in Tetrahydrofuran versetzt. Nach 1 Stunde bei Raumtemperatur wird nochmals auf 0°C gekühlt und 19.3 g Stilben-4-aldehyd zugegeben. Dann wird 2,5 Stunden bei 50°C gerührt, auf 0°C abgekühlt und der ausgefallene Feststoff abgesaugt. Der Rückstand wird mit 0.5 I THF nachgewaschen. Die organische Phase wird mit 750 ml Ethylacetat verdünnt und mit 750 ml ges. Ammoniumchlorid-Lösung gewaschen. Die Wasserphase wird mit 750 ml Essigester extrahiert, die organische Phase über Natriumsulfat getrocknet und eingeengt. Das Rohprodukt wird in der nächsten Stufe eingesetzt.
Ausbeute: 49.9 g

### Stufe 3

26.7 g Rohprodukt aus Stufe 2 wird zusammen mit 5 g Palladium auf Aktivkohle (10 % Pd) in 1000 ml Methanol suspendiert. Es wird 3 Stunden bei Raumtemperatur unter Normaldruck hydriert. Der Katalysator wird heiß abfiltriert, die Lösung im Vakuum eingeengt und das Produkt durch Chromatographie über Kieselgel mit Heptan/Essigester (10:1) gereinigt.
Ausbeute: 7.4 g

### Stufe 4

1.98 g Stufe 3 werden in 60 ml Ethanol suspendiert und mit einer Lösung von 508 mg KOH in 10 ml Wasser versetzt. Die Lösung wird 1.5 Stunden unter Rückfluß erhitzt. Das Ethanol wird im Vakuum entfernt, der Rückstand in 500 ml Essigester und 200 ml Wasser aufgenommen und die Lösung mit 2N HCI auf pH 2 gestellt. Man läßt 0,5 Stunden nachrühren, trennt die Phasen und extrahiert die Wasserphasen noch einmal mit 200 ml Ethylacetat. Die organischen Phasen werden zusammengegeben, über Natriumsulfat getrocknet und im Vakuum eingeengt.
Ausbeute: 1.86 g der Titelverbindung

### Säurechlorid:

1.23 g Stufe 4 werden in 10 ml Thionylchlorid suspendiert. Dann wird unter Rückfluß erhitzt, bis die Gasentwicklung beendet ist. Nach dem Abkühlen wird im Vakuum eingeengt und zweimal mit je 5 ml Toluol abgedampft.
Ausbeute: 1.35 g hellgraue kristalline Verbindung

### Beispiel 72

### 4-(2-(Biphenyl-4-yl)ethyl)benzoesäure

### Stufe 1:

Analog zu Stufe 2 in Beispiel 71 werden 6.4 g Phosphoniumbromid (Stufe 1 aus Beispiel 71) mit 1.82 g Biphenyl-4-aldehyd umgesetzt.
Ausbeute: 5.8 g

### Stufe 2:

5.8 g Stufe 1 werden analog zu Stufe 3 in Beispiel 71 hydriert und das Produkt durch Chromatographie gereinigt.
Ausbeute: 970 mg

### Stufe 3:

950 mg Stufe 2 werden analog zu Stufe 4 in Beispiel 71 verseift.
Ausbeute: 880 mg

### Stufe 4:

850 mg Stufe 3 werden mit Thionylchlorid analog zu Stufe 5 in Beispiel 71 zum Säurechlorid umgesetzt.
Ausbeute: 909 mg

## Patentansprüche

1. Lipopeptid A 1437-Derivate der Formel I 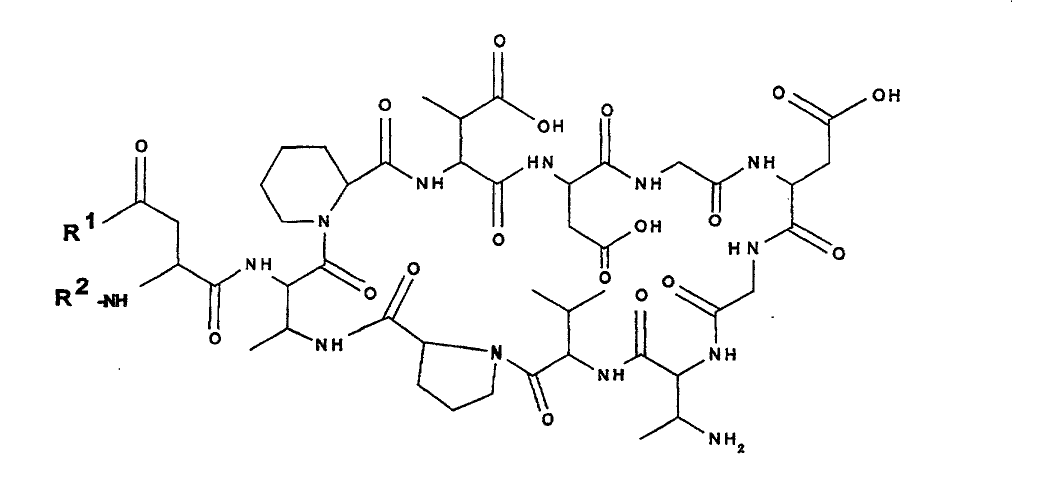 worin
R¹ OH oder NH₂,
R² ein geradkettig oder verzweigter gesättigter oder ungesättigter aliphatischer C₈-C₂₂-Acylrest ist, der durch Phenyl- oder Cycloalkylgruppen oder durch Sauerstoff unterbrochen ist
und deren pharmazeutisch verträgliche Salze.

2. Lipopeptid-Derivat gemäß Anspruch 1, **dadurch gekennzeichnet, daß** R² ein durch Phenyl- oder Cycloalkylreste unterbrochener aliphatischer Acylrest, vorzugsweise 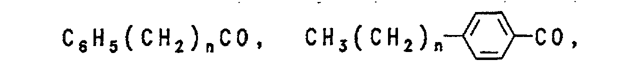 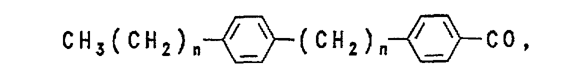 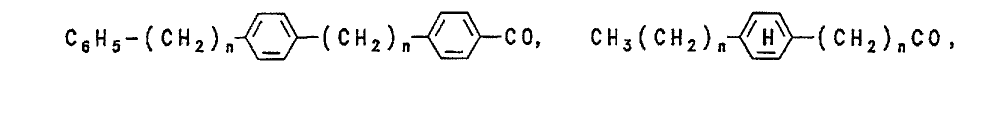 durch einen Sauerstoff unterbrochener Acylrest, vorzugsweise 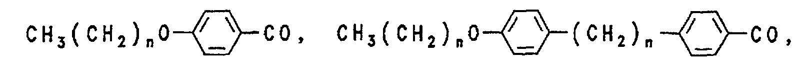 und worin n ganze Zahlen zwischen 0 und 20 bedeutet.

3. Lipopeptid-Derivat gemäß Anspruch 1, **dadurch gekennzeichnet, daß**
R¹ OH oder NH₂
R² einen durch 1-3 Phenylreste und/oder zusätzlich durch Sauerstoff unterbrochenen aliphatischen Acylrest, vorzugsweise 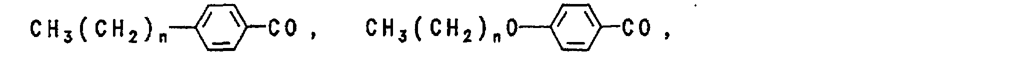 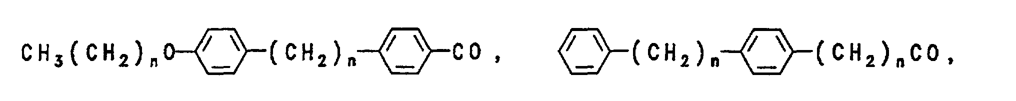 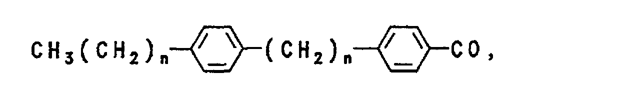 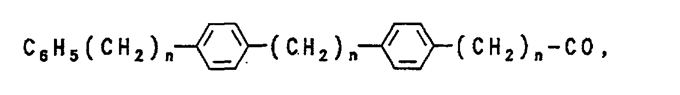
bedeutet und
worin n eine ganze Zahl zwischen 0 und 8 bedeutet.

4. Lipopeptid-Derivat gemäß Anspruch 1, **dadurch gekennzeichnet, daß**
R¹ OH oder NH₂
R² einen durch 3 Phenylgruppen unterbrochenen aliphatischen Acylrest, vorzugsweise 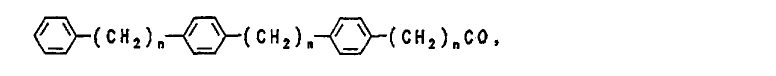 bedeutet
worin
n eine ganze Zahl zwischen 0 und 2 bedeutet.

5. Verfahren zur Herstellung einer Verbindung der Formel I, **dadurch gekennzeichnet, daß** man eine Verbindung der Formel II 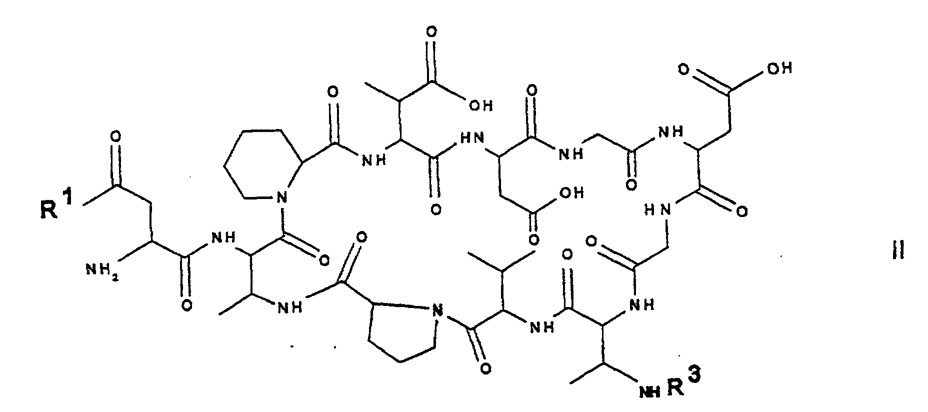 worin
R¹ die oben genannte Bedeutung hat und
R³ eine aus der Peptidchemie bekannte Aminoschutzgruppe, vorzugsweise die tert.-Butoxycarbonyl (BOC-), die Benzyloxycarbonyl- (Z-, Cbz-), die Fluorenylmethoxycarbonyl (Fmoc-) oder die Allyloxycarbonyl- (Alloc-) Schutzgruppe bedeutet,
mit einer Carbonsäure der Formel III,
R²OH III
worin
R² die in Anspruch 1 genannten Bedeutungen hat,
oder mit einem an der Carbonylgruppe aktivierten Derivat dieser Carbonsäure umsetzt.

6. Lipopeptid-Derivat gemäß Formel I zur Verwendung als Arzneimittel.

7. Arzneimittel, enthaltend Lipopeptid-Derivat gemäß Formel I sowie pharmazeutische Träger.

8. Verwendung eines Lipopeptid-Derivats gemäß Formel I zur Herstellung eines Arzneimittels gegen bakterielle Infektionen.

## Claims

1. A lipopeptide A 1437 derivative of the formula I 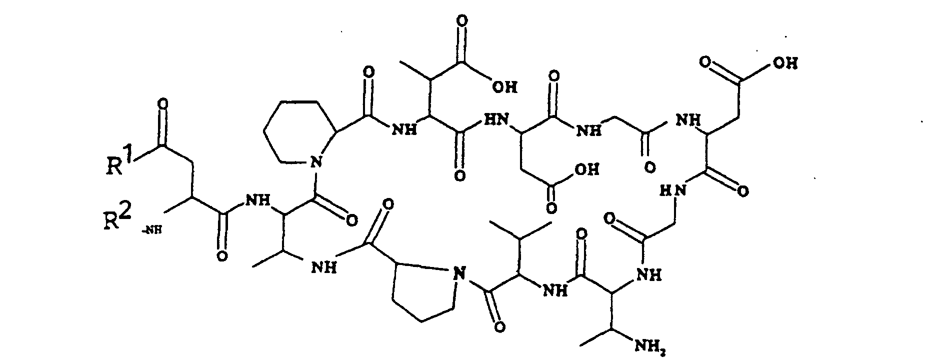 in which
R¹ is OH or NH₂,
R² is a straight-chain or branched, saturated or unsaturated aliphatic C₈-C₂₂-acyl radical which is interrupted by phenyl or cycloalkyl groups or by oxygen,
and pharmaceutically acceptable salts thereof.

2. A lipopeptide derivative as claimed in claim 1, wherein R² is an aliphatic acyl radical which is interrupted by phenyl or cycloalkyl radicals, preferably 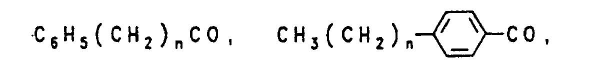 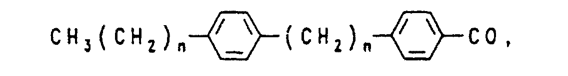 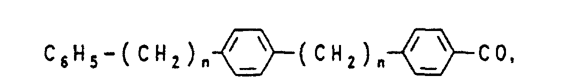 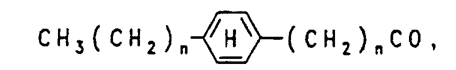 an acyl radical which is interrupted by an oxygen, preferably 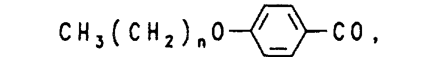 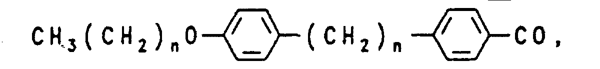 and in which n is integers between 0 and 20.

3. A lipopeptide derivative as claimed in claim 1, wherein
R¹ is OH or NH₂,
R² is an aliphatic acyl radical which is interrupted by 1-3 phenyl radicals and/or additionally by oxygen, preferably 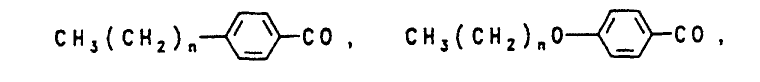 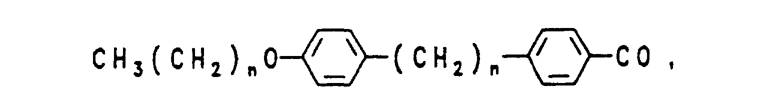 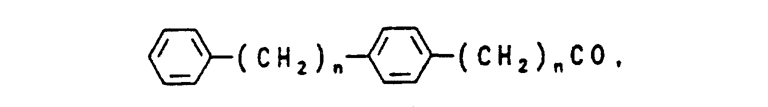 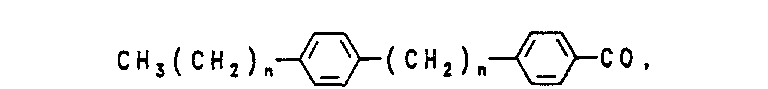 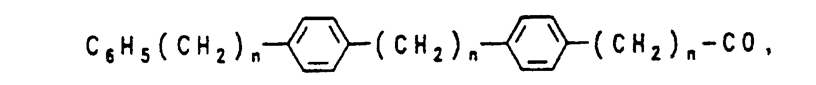
and
in which n is an integer between 0 and 8.

4. A lipopeptide derivative as claimed in claim 1, wherein
R¹ is OH or NH₂,
R² is an aliphatic acyl radical which is interrupted by 3 phenyl groups, preferably 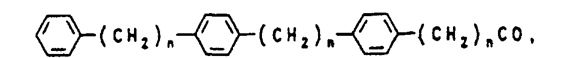
in which
n is an integer between 0 and 2.

5. A process for preparing a compound of the formula I, which comprises reacting a compound of the formula II 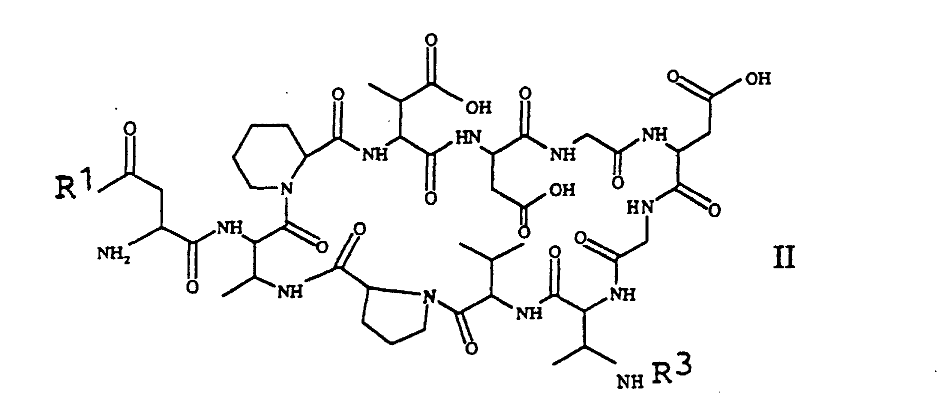 in which
R¹ has the abovementioned meaning, and
R³ is an amino protective group known from peptide chemistry, preferably the tert-butoxycarbonyl (BOC), the benzyloxycarbonyl (Z, Cbz), the fluorenylmethoxycarbonyl (Fmoc) or the allyloxycarbonyl (Alloc) protective group,
with a carboxylic acid of the formula III
R²OH III
in which
R² has the meanings specified in claim 1
or with a derivative of this carboxylic acid which is activated on the carbonyl group.

6. A lipopeptide derivative according to the formula I for use as pharmaceutical.

7. A pharmaceutical containing a lipopeptide derivative according to formula I and pharmaceutical vehicles.

8. The use of a lipopeptide derivative according to formula I for producing a pharmaceutical for bacterial infections.

## Revendications

1. Dérives du lipopeptide A 1437 de formule I 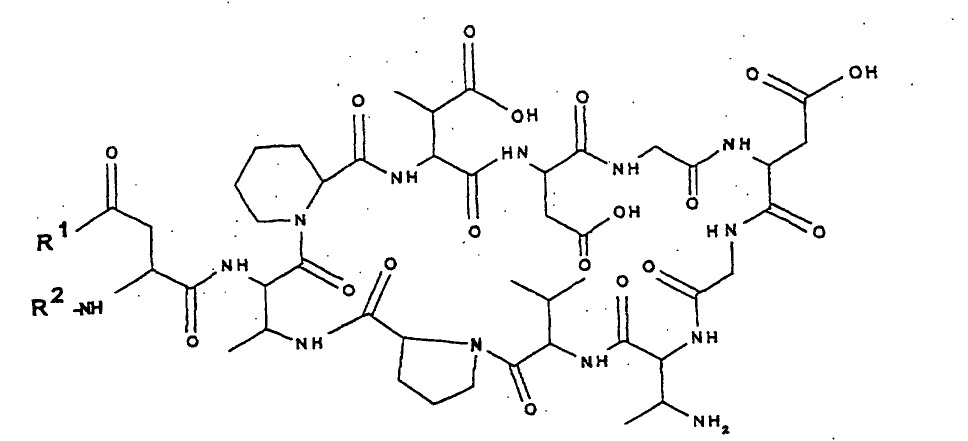 où
R¹ représente dès groupes OH ou NH₂,
R² représente un groupe acyle en C₈-C₂₂ aliphatique, linéaire ou ramifié, saturé ou insaturé, qui est interrompu par des groupes phényle ou cycloalkyle ou par des atomes d'oxygène,
et leurs sels tolérés du point de vue pharmaceutique.

2. Dérivé de lipopeptide selon la revendication 1, **caractérisé en ce que** R² représente un groupe acyle aliphatique interrompu par des groupes phényle ou cycloalkyle, de préférence 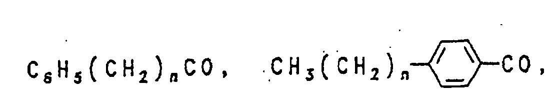 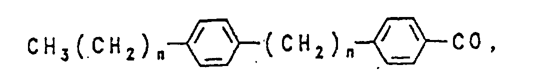 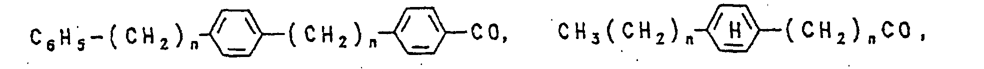 par un groupe acyle interrompu par des atomes d'oxygène, de préférence 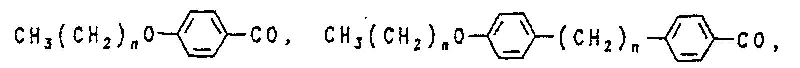 et n est un entier compris entre 0 et 20.

3. Dérivé de lipopeptide selon la revendication 1, **caractérisé en ce que**
R¹ représente des groupes OH ou NH₂,
R² représente un groupe acyle aliphatique interrompu par 1 à 3 groupes phényle et/ou de plus par des atomes d'oxygène, de préférence 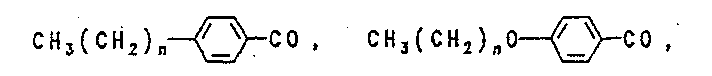 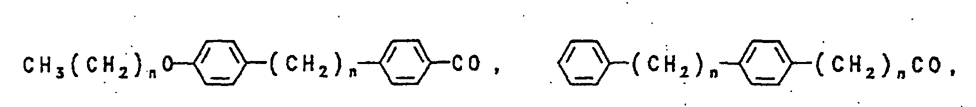 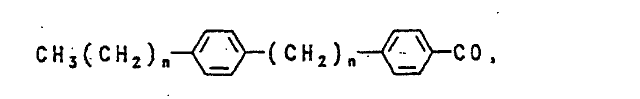 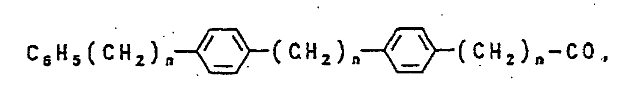
et
n est un entier compris entre 0 et 8.

4. Dérivé de lipopeptide selon la revendication 1, **caractérisé en ce que**
R¹ représente des groupes OH ou NH₂,
R² représente un groupe acyle aliphatique interrompu par 1 à 3 groupes phényle, de préférence 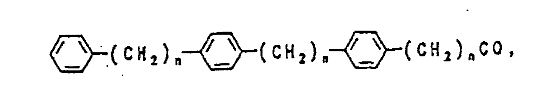
où
n est un entier compris entre 0 et 2.

5. Procédé pour la préparation d'un composé de formule I, **caractérisé en ce qu'**on fait réagir un composé de formule II 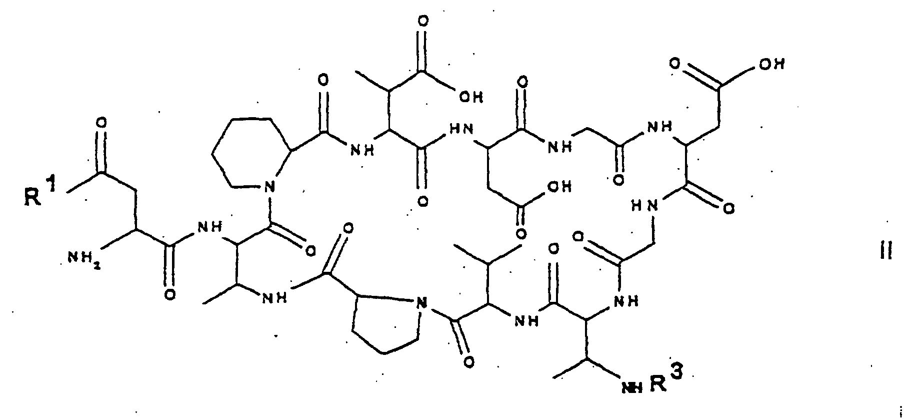 où
R¹ possède la signification donnée ci-dessus et
R³ représente un groupe protecteur d'amino connu de la chimie des peptides, de préférence les groupes protecteurs tert.-butoxycarbonyle (BOC), benzyloxycarbonyle (Z, Cbz), fluorénylméthoxycarbonyle (Fmoc) ou allyloxycarbonyle (Alloc),
sur un acide carboxylique de formule III
R²OH III
où
R² possède les significations données à la revendication 1,
ou est substitué par un dérivé activé sur le groupe carbonyle de cet acide carboxylique.

6. Dérivé de lipopeptide selon la formule I pour l'utilisation comme médicament.

7. Médicament contenant un dérivé de lipopeptide de formule I, ainsi que des supports pharmaceutiques.

8. Utilisation d'un dérivé de lipopeptide de formule I pour la fabrication d'un medicament contre les infections bactériennes.
